(19) Europäisches Patentamt
European Patent Office
Office européen des brevets

(11) Veröffentlichungsnummer: **0 168 765 B1**

(12) # EUROPÄISCHE PATENTSCHRIFT

(45) Veröffentlichungstag der Patentschrift: **16.08.89**

(51) Int. Cl.⁴: **C 07 D 253/06**

(21) Anmeldenummer: **85108576.1**

(22) Anmeldetag: **10.07.85**

(54) **Verfahren zur Herstellung S-substituierter Isothioharnstoffe.**

(30) Priorität: **18.07.84 US 632136**

(43) Veröffentlichungstag der Anmeldung: **22.01.86 Patentblatt 86/4**

(45) Bekanntmachung des Hinweises auf die Patenterteilung: **16.08.89 Patentblatt 89/33**

(84) Benannte Vertragsstaaten: **AT BE CH DE FR GB IT LI NL**

(56) Entgegenhaltungen:
**FR-A- 1 577 658**
**FR-A- 2 233 325**
**GB-A- 1 542 444**

**Die Akte enthält technische Angaben, die nach dem Eingang der Anmeldung eingereicht wurden und die nicht in dieser Patentschrift enthalten sind.**

(73) Patentinhaber: **MOBAY CORPORATION, Mobay Road, Pittsburgh Pennsylvania 15205-9741 (US)**

(72) Erfinder: **Jackman, Dennis E., 7350 Roe Circle, Prairie Village Kansas 66208 (US)**
Erfinder: **Westphal, Dietmar B., Dr., 8958 Woodstone, Lenexa Kansas 66219 (US)**

(74) Vertreter: **Schumacher, Günter, Dr. et al, c/o Bayer AG Konzernverwaltung RP Patentabteilung, D-5090 Leverkusen 1 Bayerwerk (DE)**

**EP 0 168 765 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein zweistufiges Verfahren zur Herstellung von in 3-Stellung durch den Rest $-SR^1$ substituierten 1,2,4-Triazin-5(4H)-on-Derivaten (wobei $R^1$ z. B. für bestimmte Alkylreste steht), ausgehend von den entsprechenden 3-Thioxo- bzw. den tautomeren 3-Thiol-Verbindungen.

Die US-A 3 671 523 offenbart das ausserordentlich wirksame selektive Herbizid der Formel (I)

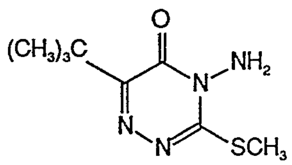

(I).

Die Verbindung wird durch Methylieren der entsprechenden Verbindung mit einem -SH-Rest in der 3-Stellung mit Hilfe eines Methylierungsmittels wie Methylbromid oder Methyliodid hergestellt, wobei Methylchlorid viel niedrigere Ausbeuten liefert. Wenngleich das Verfahren gut arbeitet (wobei jedoch teilweise eine Methylierung am Stickstoff-Atom in der 2-Stellung stattfindet), sind Methylbromid und -iodid doch relativ teuer.

Die US-A 4 457 774 offenbart ein ähnliches Verfahren zur Herstellung der Verbindung (II)

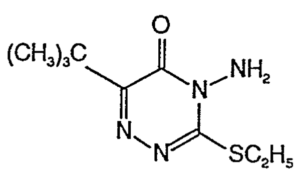

(II),

die ebenfalls selektiv herbizid wirkt, wobei Ethylbromid oder -iodid zur Verwendung gelangen, die noch teurer sind als Methylbromid bzw. -iodid und auch noch geringere Produktausbeuten liefern.

Der Stand der Technik umfasst die Offenbarung mehrerer anderer Triazinone, die einen S-Alkyl-Substituenten in der 3-Stellung und andere Substituenten in der 4-Stellung und der 6-Stellung tragen; stellt man jedoch praktische wirtschaftliche Betrachtungen an, so werden diese Verbindungen unter Verwendung teurer Alkylierungsmittel hergestellt, die mit den entsprechenden 3-Thiolen zur Reaktion gebracht werden.

Dementsprechend ist es ein Ziel der vorliegenden Erfindung, 1,2,4-Triazin-5(4H)-on-Derivate mit einem $-SR^1$-Rest in 3-Stellung in einfacher und preisgünstiger Weise aus den entsprechenden 3-Thioxo-Verbindungen bzw. den tautomeren 3-Thiolen herzustellen.

Gegenstand der Erfindung ist ein Verfahren zur Herstellung von in 3-Stellung durch den Rest $-SR^1$ substituierten 1,2,4-Triazin-5(4H)-on-Derivaten der allgemeinen Formel (III)

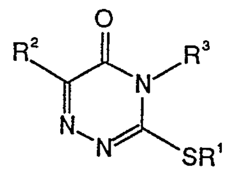

(III),

in welcher

$R^1$ ein jeweils gegebenenfalls substituierter Alkyl-, Alkenyl-, Phenyl- oder Phenylalkyl-Rest mit bis zu 12 Kohlenstoff-Atomen in den aliphatischen Resten ist und

$R^2$ und $R^3$ die nachfolgend angegebenen Bedeutungen haben,

durch Umsetzung von 3-Thioxo-5-oxo-tetrahydro-1,2,4(2H,4H)-triazinen der allgemeinen Formel (IV)

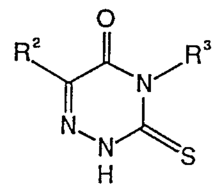

(IV),

in welcher

$R^2$ ein gegebenenfalls halogen-substituierter Alkyl- oder Alkenyl-Rest mit jeweils bis zu 12 Kohlenstoff-Atomen oder ein Phenyl-Rest ist und

$R^3$ ein $NH_2$-Rest oder ein Alkyl-, Alkenyl-, Aryl-, Aralkyl-, Alkylamino-, Dialkylamino-, Alkenylamino- oder Arylamino-Rest mit jeweils bis zu 12 Kohlenstoff-Atomen ist,

mit einem Veretherungsmittel zum Ersatz von H durch $R^1$, welches dadurch gekennzeichnet ist, dass die Reaktion in zwei Schritten durchgeführt wird, wobei in dem ersten Schritt die 3-Thioxo-5-oxo-tetrahydro-1,2,4-(2H,4H)-triazine mit einem alpha-aktivierten Veretherungsmittel der Formel (V)

X–A–Y (V),

in welcher

X ein Halogenatom ist,

A eine direkte Bindung oder $CH_2$ ist und

Y eine Elektronen abziehende Gruppe ist,

unter Verwendung von Natriumhydroxid und in Anwesenheit eines Lösungsmittels zu den Zwischenprodukten der Formel (VI)

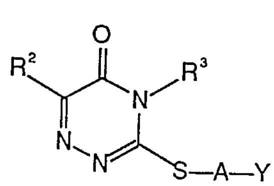

(VI),

umgesetzt werden und diese Zwischenprodukte in dem zweiten Schritt unter den gleichen Reaktionsbedingungen – in Gegenwart von Natriumhydroxid und in Anwesenheit eines Lösungsmittels – mit einer Verbindung der Formel $R^1$-SH umgesetzt werden (wobei als Nebenprodukte Verbindungen der Formel HS–A–Y gebildet werden).

Die Reaktionsfolge sei wie folgt schematisch veranschaulicht:

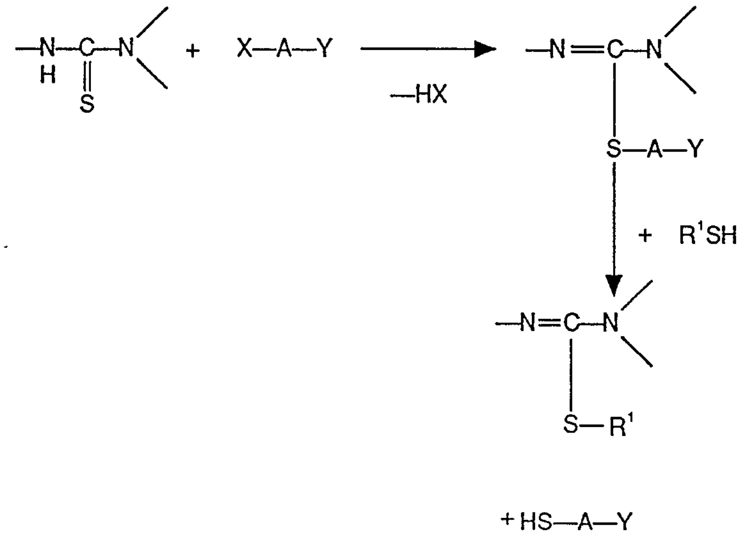


In der allgemeinen Formel X–A–Y bezeichnen X ein Halogenid, insbesondere das Chlorid oder Bromid, A eine direkte Bindung oder, vorzugsweise, $CH_2$ und Y eine aktivierende (Elektronen abziehende) Gruppe wie Carboxyl, Acyl, Nitro, Cyano, Sulfonyl, Sulfinyl, Trifluoromethyl oder einen anderen elektronisch ähnlichen Rest, und vorteilhafterweise ist in den obigen Formeln $R^1$ ein jeweils gegebenenfalls substituierter Alkyl-, Alkenyl-, Phenyl- oder Phenylalkyl-Rest, wobei die aliphatischen Reste bis zu 12 und insbesondere bis zu 4 Kohlenstoff-Atome enthalten. Als spezielles Beispiel für $R^1$ SH sei Ethylmercaptan genannt.

Das Verfahren ist anwendbar speziell bei Ausgangsmaterialien, in denen
$R^2$ verzweigtes, gegebenenfalls halogen-substituiertes Alkyl mit bis zu 6 Kohlenstoff-Atomen ist, z.B. tert.-Butyl, Fluor- oder Chlor-tert-butyl und verzweigtes, ein quaternäres Kohlenstoffatom enthaltendes Pentyl, und
$R^3$ $NH_2$ oder Alkyl, Alkylamino oder Dialkylamino mit jeweils bis zu 4 Kohlenstoff-Atomen ist, insbesondere $NH_2$ oder Methyl.

Vorteilhafterweise werden beide Schritte der Reaktion in einem Lösungsmittel und in Gegenwart eines Katalysators sowie unter Entfernung des Nebenprodukts HSAY durch Extraktion durchgeführt.

Die Reaktionstemperatur kann von Raumtemperatur oder noch tieferer Temperatur bis zu Siedetemperatur, je nach dem verwendeten Lösungsmittel, reichen, beträgt vorzugsweise jedoch 30 °C bis 100 °C.

Zu geeigneten Lösungsmitteln zählen inerte organische Lösungsmittel wie Toluol, halogenierte Kohlenwasserstoffe oder dergleichen, jedoch wird der Einsatz von Wasser bevorzugt, wenn das Reagens oder das Endprodukt wasserlöslich ist, z. B. Chloressigsäure in Gegenwart von Alkali.

Obwohl die Reaktionspartner in stöchiometrischen Mengen eingesetzt werden können, können auch grosse Überschüsse des einen der beiden Reaktionspartner eingesetzt werden, vorzugsweise des billigeren Reaktionsteilnehmers, um die Reaktion zur Vervollständigung zu treiben.

Die Reaktionen unter Einsatz von Chloressigsäure als Veretherungsmittel der Formel X–A–Y lassen sich wiederum schematisch wie folgt darstellen:

Schritt 1

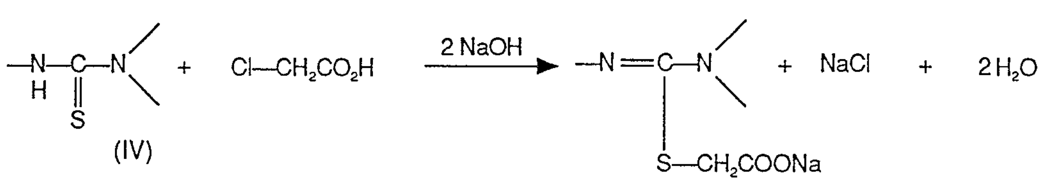


(IV)

(VII)

Schritt 2

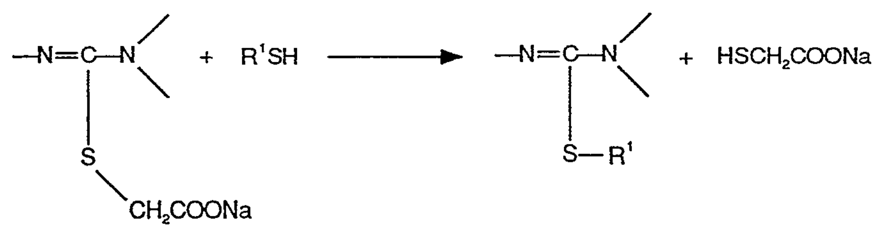

(VII) (III) (VIII)

In Schritt 1 werden die Chloressigsäure in das Na-Salz und das Thiol (IV) zunächst in das Na-Salz und anschliessend in das Thiolessigsäurederivat (VII) überführt. In Schritt 2 reagiert (VII) (wasserlöslich) mit $R^1SH$ (wasserunlöslich) zu (III) (wasserunlöslich, löslich in überschüssigem $R^1SH$) und (VIII) (wasserlöslich). Die Reaktion ist reversibel, schreitet jedoch in der angegebenen Richtung fort, da (III) und (VIII) verschiedene Löslichkeiten besitzen. (VIII) wird durch Extraktion in das Wasser hinein entfernt.

Die Reaktion benutzt 2 Äquivalente NaOH und vorzugsweise mehr (etwa 0,3 mol), um in Schritt 2 einen pH von 9 aufrechtzuerhalten. Dies ist zwar nicht erforderlich, um die Reaktion ablaufen zu lassen, aber es ist von Vorteil für die Ausbeute und die Reinheit. In diesem Fall ist das Nebenprodukt (VIII) tatsächlich eine Mischung aus $HSCH_2COONa$ und $NaSCH_2COONa$. Dieser höhere pH hilft auch der Reaktion, da ein Teil des $R^1SH$ in $R^1SNa$ überführt wird, was wahrscheinlich die Species ist, die (VII) angreift und zur Bildung der Produkte führt.

Eine Vielzahl verschiedenartiger alpha-aktivierter Halogenide kann zur Bildung des Zwischenproduktes (VII) in dem ersten Schritt herangezogen werden, z. B. Cyanchlorid, Bromnitromethan, Chlordimethylsulfon, Chloraceton, 4-Nitrobenzylchlorid, 2,4-Dinitrobenzylchlorid und dergleichen.

Bromessigsäure und insbesondere Chloressigsäure werden jedoch in hohem Masse bevorzugt. Der Grund liegt darin, dass sie sich bei der Veretherung im Vergleich zu anderen Mitteln, die in unerwünschter Weise das Stickstoff-Atom in 2-Stellung in wechselndem Ausmass verethern, als besonders selektiv erwiesen haben. Beispielsweise sind in der ersten Stufe der Veretherung die Prozentsätze der unerwünschten N-Alkylierung (der Rest zu 100% stellt die erwünschte S-Alkylierung dar) durch verschiedene Alkylierungsmittel der foglenden Tabelle zu entnehmen:

| Alkylierungsmittel | % N-Alkylierung |
|---|---|
| $Br-CH_2-CH_3$ | 16 |
| $Br-CH_2-H$ | 6 |
| $Br-CH_2-CO_2H$ | 0 |
| $Cl-CH_2-CH_3$ | 80-90 |
| $Cl-CH_2-H$ | 30-40 |
| $Cl-CH_2-CO_2H$ | 0 |

Die Reaktion des aktivierten Chlorids mit der 3-Thioxo-Verbindung kann gewünschtenfalls in wässriger Lösung durchgeführt werden, wobei möglichst der pH so eingestellt wird, dass Löslichkeit, z. B. der Chloressigsäure in alkalischer Lösung, erzielt wird. Die Reaktion kann durch Anwesenheit einer kleinen Menge Natriumbromid beschleunigt werden, das offenbar katalytisch wirkt und in jedem passenden Stadium zurückgewonnen werden kann, z. B. nach der Veretherung oder nach der Umetherung.

Die vorliegende Erfindung ist besonders wertvoll, wenngleich nicht auf einen solchen Einsatz beschränkt, für die Herstellung der nachstehenden Verbindungen neben derjenigen, die im Folgenden beispielhaft genannt wird, und denjenigen, die in der gleichzeitig eingereichten Anmeldung (EP-Anmeldung 85 108 577.9-2101/Veröffentlichungsnummer 0 168 766) beschrieben werden, auf die hier Bezug genommen wird:

4-Amino-3-methylthio-6-tert-butyl-1,2,4-triazin-5-on,
4-Amino-3-ethylthio-6-tert-butyl-1,2,4-triazin-5-on,
4-Amino-6-fluoro-tert-butyl-3-allylthio-1,2,4-triazin-5-on,
4-Amino-6-fluoro-tert-butyl-3-methylthio-1,2,4-triazin-5-on,
4-Amino-6-(1,1-bis-fluoromethyl-ethyl)-3-buten-2-yl-thio-1,2,4-triazin-5-on,
4-Amino-6-ethoxy-tert-butyl-3-propargylthio-1,2,4-triazin-5-on,
6-(2,3-Dimethyl-but-2-yl)-4-methyl-3-methylthio-1,2,4-triazin-5-on.

Das nachfolgende Beispiel dient zur weiteren Erläuterung der Erfindung.

Beispiel

Eine Mischung aus 200 g (1 mol) 4-Amino-6-tert-butyl-3-mercapto-1,2,4-triazin-5-on, 200 ml Wasser, 20,6 g (0,2 mol) Natriumbromid und 108 g (1,14 mol) Chloressigsäure wurden unter Rühren mit 25-proz. NaOH behandelt, bis der pH den Wert 9,5 erreichte. Die Lösung wurde dann unter Rühren auf 50 °C erhitzt, wobei der pH auf 9,5 gehalten wurde, bis die Reaktion vollständig war (gewöhnlich 3 bis 5 h). Nach dem Abkühlen auf eine Temperatur unterhalb von 30 °C wurde Ethylmercaptan (372 g: 6 mol) hinzugefügt, und die Mischung wurde unter Rühren bei einem pH

von 9 bis 9,5 zum Rückfluss erhitzt, bis das Zwischenprodukt 4-Amino-6-tert-butyl-3-carboxymethylthio-1,2,4-triazin-5-on vollständig umgesetzt worden war (Dünnschichtchromatographie), wobei die Zeitspanne dafür gewöhnlich 3 bis 5 h betrug. Die obere organische Schicht wurde dann entfernt, mit 200 bis 300 ml Wasser gemischt, und das überschüssige Ethylmercaptan wurde durch Destillation zurückgewonnen. Die wässrige Suspension von 4-Amino-6-tert-butyl-3-ethylthio-1,2,4-triazin-5-on wurde filtriert, wonach das Produkt in einer Ausbeute von etwa 95% mit einer Reinheit von 98% erhalten wurde.

**Patentansprüche**

1. Verfahren zur Herstellung von in 3-Stellung durch den Rest $-SR^1$ substituierten 1,2,4-Triazin-5(4H)-on-Derivaten der allgemeinen Formel (III)

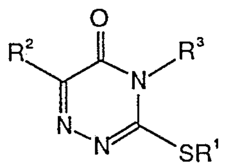

(III),

in welcher
$R^1$ ein jeweils gegebenenfalls substituierter Alkyl-, Alkenyl-, Phenyl- oder Phenylalkyl-Rest mit bis zu 12 Kohlenstoff-Atomen in den aliphatischen Resten ist und
$R^2$ und $R^3$ die nachfolgend angegebenen Bedeutungen haben,
durch Umsetzung von 3-Thioxo-5-oxo-tetrahydro-1,2,4(2H,4H)-triazinen der allgemeinen Formel (IV)

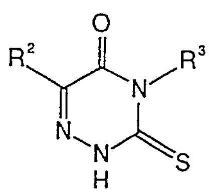

(IV),

in welcher
$R^2$ ein gegebenenfalls halogen-substituierter Alkyl- oder Alkenyl-Rest mit jeweils bis zu 12 Kohlenstoff-Atomen oder ein Phenyl-Rest ist und
$R^3$ ein $NH_2$-Rest oder ein Alkyl-, Alkenyl-, Aryl-, Aralkyl-, Alkylamino-, Dialkylamino-, Alkenylamino- oder Arylamino-Rest mit jeweils bis zu 12 Kohlenstoff-Atomen ist,
mit einem Veretherungsmittel zum Ersatz von H durch $R^1$, dadurch gekennzeichnet, dass die Reaktion in zwei Schritten durchgeführt wird, wobei in dem ersten Schritt die 3-Thioxo-5-oxo-tetrahydro-1,2,4-(2H,4H)-triazine mit einem alpha-aktivierten Veretherungsmittel der Formel (V)

$$X—A—Y \quad (V)$$

in welcher
X ein Halogenatom ist,
A eine direkte Bindung oder $CH_2$ ist und
Y eine Elektronen abziehende Gruppe ist,
unter Verwendung von Natriumhydroxid und in Anwesenheit eines Lösungsmittels zu den Zwischenprodukten der Formel (VI)

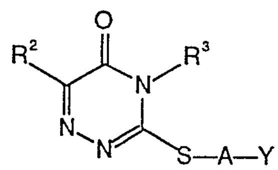

(VI),

umgesetzt werden und diese Zwischenprodukte in dem zweiten Schritt unter den gleichen Reaktionsbedingungen – in Gegenwart von Natriumhydroxid und in Anwesenheit eines Lösungsmittels – mit einer Verbindung der Formel $R^1$-SH umgesetzt werden.

2. Verfahren nach Anspruch 1, dadurch gekennzeichnet, dass das Veretherungsmittel der Formel X–A–Y in dem ersten Schritt ein alpha-aktiviertes Alkylhalogenid ist.

3. Verfahren nach Anspruch 2, dadurch gekennzeichnet, dass X–A–Y Chloressigsäure oder Bromessigsäure ist.

4. Verfahren nach Anspruch 3, dadurch gekennzeichnet, dass die Umsetzung in Anwesenheit von Wasser als Lösungsmittel durchgeführt wird.

5. Verfahren nach Anspruch 4, dadurch gekennzeichnet, dass Natriumbromid als Katalysator anwesend ist.

6. Verfahren nach Anspruch 4, dadurch gekennzeichnet, dass während des zweiten Schrittes oder nach dem zweiten Schritt der Reaktion das Nebenprodukt der Formel HS–A–Y aus dem Hauptprodukt durch Extraktion in Wasser hinein entfernt wird.

7. Verfahren nach Anspruch 1, dadurch gekennzeichnet, dass
$R^1$ ein Alkyl-, Alkenyl-, Phenyl- oder Phenylalkyl-Rest mit bis zu 4 Kohlenstoff-Atomen in den aliphatischen Resten ist.

8. Verfahren nach Anspruch 1, dadurch gekennzeichnet, dass
$R^2$ verzweigtes, gegebenenfalls halogen-substituiertes Alkyl mit bis zu 6 Kohlenstoff-Atomen ist und
$R^3$ $NH_2$ oder Alkyl, Alkylamino oder Dialkylamino mit jeweils bis zu 4 Kohlenstoff-Atomen ist.

9. Verfahren nach Ansprüchen 7 und 8, dadurch gekennzeichnet, dass
$R^1$ $C_2H_5$ ist,
$R^2$ $C(CH_3)_3$ ist und
$R^3$ $NH_2$ ist.

**Claims**

1. Process for the preparation of 1,2,4-triazin-5(4H)-one derivates which are substituted in the 3-position by the radical $-SR^1$, of the general formula (III)

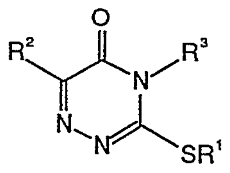

(III),

in which

$R^1$ is an alkyl, alkenyl, phenyl or phenylalkyl radical which has up to 12 carbon atoms in the aliphatic radicals and which is in each case optionally substituted and

$R^2$ and $R^3$ have the meanings indicated below, by reacting 3-thioxo-5-oxo-tetrahydro-1,2,4(2H,4H)-triazines, of the general formula (IV)

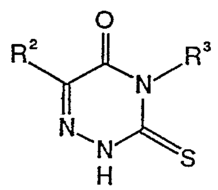

(IV)

in which

$R^2$ is an optionally halogen-substituted alkyl or alkenyl radical each of which has up to 12 carbon atoms or a phenyl radical and

$R^3$ is an $NH_2$ radical or an alkyl, alkenyl, aryl, aralkyl, alkylamino, dialkylamino, alkenylamino or arylamino radical each of which has up to 12 carbon atoms

with an etherifying agent in order to replace H by $R^1$, characterized in that the reaction is carried out in two steps, where in the first step the 3-thioxo-5-oxo-tetrahydro-1,2,4-(2H, 4H)-triazines are reacted with an alpha-activated etherifying agent of the formula (V),

X–A–Y (V),

in which

X is a halogen atom,

A is a direct bond or $CH_2$ and

Y is an electron-withdrawing group,

with the use of sodium hydroxide and in the presence of a solvent, to give the intermediates of the formula (VI)

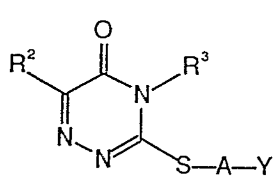

(VI),

and these intermediates are reacted in the second step under identical reaction conditions – in the presence of sodium hydroxide and in the presence of a solvent – with a compound of the formula $R^1$-SH.

2. Process according to Claim 1, characterized in that the etherifying agent of the formula X–A–Y in the first step is an alpha-activated alkyl halide.

3. Process according to Claim 2, characterized in that X–A–Y is chloroacetic acid or bromoacetic acid.

4. Process according to Claim 3, characterized in that the reaction is carried out in the presence of water as the solvent.

5. Process according to Claim 4, characterized in that sodium bromide is present as the catalyst.

6. Process according to Claim 4, characterized in that during the second step or after the second step of the reaction the by-product of the formula HS–A–Y is removed from the main product by extraction into water.

7. Process according to Claim 1, characterized in that

$R^1$ is an alkyl, alkenyl, phenyl or phenylalkyl radical having up to 4 carbon atoms in the aliphatic radicals.

8. Process according to Claim 1, characterized in that

$R^2$ is a branched, optionally halogen-substituted alkyl having up to 6 carbon atoms and

$R^3$ ins $NH_2$ or alkyl, alkylamino or dialkylamino each of which has up to 4 carbon atoms.

9. Process according to Claims 7 and 8, characterized in that

$R^1$ is $C_2H_5$,

$R^2$ is $C(CH_3)_3$ and

$R^3$ is $NH_2$.

## Revendications

1. Procédé de préparation de dérivés de 1,2,4-triazin-5(4H)-ones substitués en position 3 par le radical $-SR^1$ et répondant à la formule générale (III):

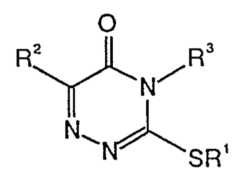

(III),

dans laquelle

$R^1$ représente un radical alkyle, un radical alcényle, un radical phényle ou un radical phénylalkyle chacun éventuellement substitué et contenant jusqu'à 12 atomes de carbone dans les radicaux aliphatiques, et

$R^2$ et $R^3$ ont les significations indiquées ci-après, par réaction de 3-thioxo-5-oxo-tétrahydro-1,2,4-(2H,4H)-triazines de formule générale (IV):

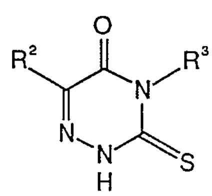

(IV),

dans laquelle

$R^2$ représente un radical alkyle ou un radical alcényle éventuellement substitué par un atome d'halogène et contenant chacun jusqu'à 12 atomes de carbone, ou un radical phényle, et
$R^3$ représente un radical $NH_2$ ou un radical alkyle, un radical alcényle, un radical aryle, un radical aralkyle, un radical alkylamino, un radical dialkylamino, un radical alcénylamino ou un radical arylamino contenant chacun jusqu'à 12 atomes de carbone, avec un agent d'éthérification en vue de remplacer H par $R^1$, caractérisé en ce qu'on effectue la réaction en deux étapes au cours de la première desquelles on fait réagir les 3-thioxo-5-oxo-tétrahydro-1,2,4-(2H,4H)-triazines avec un agent d'éthérification alpha-activé de formule (V):

X-A-Y (V)

dans laquelle
X représente un atome d'halogène,
A représente une liaison directe ou $CH_2$, et
Y représente un groupe éliminant des électrons, en utilisant de l'hydroxyde de sodium et en présence d'un solvant, pour obtenir les produits intermédiaires de formule (VI):

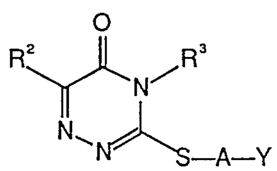

(VI),

et, lors de la deuxième étape, dans les mêmes conditions réactionnelles (en présence d'hydroxyde de sodium et en présence d'un solvant), on fait réagir ces produits intermédiaires avec un composé de formule $R^1$-SH.

2. Procédé selon la revendication 1, caractérisé en ce que l'agent d'éthérification de formule X-A-Y intervenant lors de la première étape est un halogénure d'alkyle alpha-activé.

3. Procédé selon la revendication 2, caractérisé en ce que X-A-Y est l'acide chloracétique ou l'acide bromacétique.

4. Procédé selon la revendication 3, caractérisé en ce qu'on effectue la réaction en présence d'eau comme solvant.

5. Procédé selon la revendication 4, caractérisé en ce que le bromure de sodium est présent comme catalyseur.

6. Procédé selon la revendication 4, caractérisé en ce que, pendant ou après la deuxième étape de la réaction, on élimine le sousproduit de formule HS-A-Y du produit principal par extraction dans l'eau.

7. Procédé selon la revendication 1, caractérisé en ce que
$R^1$ représente un radical alkyle, un radical alcényle, un radical phényle ou un radical phénylalkyle contenant jusqu'à 4 atomes de carbone dans les radicaux aliphatiques.

8. Procédé selon la revendication 1, caractérisé en ce que
$R^2$ représente un radical alkyle ramifié, éventuellement substitué par un atome d'halogène et contenant jusqu'à 6 atomes de carbone, et
$R^3$ représente $NH_2$ ou un radical alkyle, un radical alkylamino ou un radical dialkylamino contenant chacun jusqu'à 4 atomes de carbone.

9. Procédé selon les revendications 7 et 8, caractérisé en ce que
$R^1$ représente $C_2H_5$,
$R^2$ représente $C(CH_3)_3$, et
$R^3$ représente $NH_2$.